(19) Europäisches Patentamt
European Patent Office
Office européen des brevets

(11) **EP 1 400 805 B1**

(12) **EUROPÄISCHE PATENTSCHRIFT**

(45) Veröffentlichungstag und Bekanntmachung des Hinweises auf die Patenterteilung:
**09.11.2016 Patentblatt 2016/45**

(21) Anmeldenummer: **03021229.4**

(22) Anmeldetag: **18.09.2003**

(51) Int Cl.:
*G01N 33/543* *(2006.01)*  *G01N 33/68* *(2006.01)*
*G01N 33/50* *(2006.01)*  *C12Q 1/02* *(2006.01)*
*G01N 27/414* *(2006.01)*

(54) **Messvorrichtung und -verfahren für ein Screening bei auf Elektroden immobilisierten Zellen**

Measuring apparatus and method for screening of cells immobilized on electrodes

Appareil de mesure et méthode pour le criblage des cellules immobilisées sur des électrodes

(84) Benannte Vertragsstaaten:
**AT BE BG CH CY CZ DE DK EE ES FI FR GB GR HU IE IT LI LU MC NL PT RO SE SI SK TR**

(30) Priorität: **19.09.2002 DE 10243599**

(43) Veröffentlichungstag der Anmeldung:
**24.03.2004 Patentblatt 2004/13**

(73) Patentinhaber: **Max-Planck-Gesellschaft zur Förderung
der Wissenschaften e.V.
80539 München (DE)**

(72) Erfinder:
• **Fromherz, Peter Prof. Dr.
80686 München (DE)**
• **Brittinger, Matthias
82234 Wessling (DE)**

(74) Vertreter: **Müller Hoffmann & Partner
Patentanwälte mbB
St.-Martin-Strasse 58
81541 München (DE)**

(56) Entgegenhaltungen:
WO-A-01/70002  WO-A-01/94375
US-A- 5 827 655  US-A- 6 051 422
US-A1- 2002 086 298

• BAUMANN W H ET AL: "Microelectronic sensor system for microphysiological application on living cells" SENSORS AND ACTUATORS B, ELSEVIER SEQUOIA S.A., LAUSANNE, CH, Bd. 55, Nr. 1, 25. April 1999 (1999-04-25), Seiten 77-89, XP004175066 ISSN: 0925-4005

**Beschreibung**

Die vorliegende Erfindung betrifft ein Verfahren und ein System für ein Screening bei auf Elektroden immobilisierten Zellen unter Verwendung eines Niedrigsalz-Mediums. Insbesondere wird ein Verfahren offenbart zur Bestimmung, ob eine Substanz ein Modulator einer Zielkomponente in einer auf einer Elektrode immobilisierten Zelle ist.

Die US 5,827,655 A beschreibt eine Probe, ein Verfahren und Produkte basierend auf $NK^+$-Kanalexpression. Elektrophysiologische Experimente werden an Ooyzten mit 2-Elektroden-Voltage-Clamp- und Patch-Clamp-Verfahren durchgeführt. Alle Experimente werden bei Raumtemperatur durchgeführt. Für das 2-Elektroden-Voltage-Clamp-Experiment werden Zellen in einer Oozyten-Ringer-Lösung gebadet, die 96 mM NaCl. 1,8 mM $CaCl_2$, 1 mM $MgCl_2$ enthält. Die Pipettenlösung ist 3 M KCl. Patch-Clamp-Experimente wurden unter identischen Bedingungen in Oozyten und T-Zellen durchgeführt, unter Verwendung einer Säugetier-Ringer-Lösung, die 160 mM NaCl, 4,5 mM KCl, 2 mM $CaCl_2$, 1 mM $M_9Cl_2$, 5 mM Hepes; pH 7,4 enthält.

Die WO 01/94375 A1 beschreibt einen Calcium-abhängigen Kaliumkanal mit hoher Leitfähigkeit als Modulator von Alkoholeffekten und Verbrauch. Hierbei wird der BK-Kanal als Ziel für Drogen, die Effekte von Alkohol als auch von Alkoholkonsum modulieren, untersucht. Ferner wird der Gebrauch von Modulatoren des BK-Kanals zur Modulation des Alkoholkonsums und des Effekts von Alkohol untersucht. Hierbei werden elektrophysiologische Messungen mittels 2-Elektroden-Voltage-Clamp-Verfahren an Oozyten durchgeführt.

Die US 2002/0086298 A1 betrifft einen G-Protein-aktivierten Säugetier-Kalium KGA-Kanal. Hierbei werden elektrophysiologische Messungen an Oozyten durchgeführt, in welchen der entsprechende Kanal exprimiert ist. Die Oozyten werden gewöhnlicherweise in einer ND96-Lösung mit 96 mM NaCl, 2 mM KCl, 1 mM $MgCl_2$, 1 mM $CaCl_2$, 5 mM Hepes und pH 7,5 gehalten.

Um pharmazeutische Effekte von Testsubstanzen zu bestimmen, werden oft sogenannte zelluläre Screening-Verfahren verwendet. Darin werden lebende Zellen in kontakt mit den Testsubstanzen gebracht, um deren Funktion als möglicher Effektor auf Zielkomponenten, z.B. Rezeptoren oder/und Ionenkanälen, in einer Zelle zu bestimmen. Hierzu wird in der internationalen Patentanmeldung PCT/EP01/03209 vorgeschlagen, ein System zu verwenden, bei dem die Wirkung einer Testsubstanz auf die Aktivität einer Zelle durch einen darunter liegenden Feldeffekt-Transistor gemessen wird.

Messungen von Analyten unter Verwendung von auf Chips immobilisierten lebenden Zellen werden auch in WO 96/31774, US-PS 6,051,422, Baumann et al. (Sensors and Activators B55 (1999), 77-89) und Fertig et al. (Biophys. J. 82 (2002), 3056-3062) beschrieben. Problematisch an derartigen Messeinrichtungen ist jedoch, dass oftmals nur ein geringes Messsignal an der mit der Zelle in kontakt stehenden Elektrode erhalten wird.

Eine Aufgabe der vorliegenden Erfindung besteht darin, eine Verbesserung bekannter Messeinrichtungen bzw. Messverfahren bereitzustellen, durch welche einerseits das Messsignal erhöht und andererseits eine neue Messmethode bei auf Elektroden immobilisierten Zellen ermöglicht wird.

Diese Aufgabe wird durch den Gegenstand der unabhängigen Ansprüche gelöst. Vorteilhafte Ausgestaltungen und Weiterbildungen der Erfindung sind in den Unteransprüchen angegeben.

Insbesondere wird eine Messung in einem Medium durchgeführt, das einen erhöhten Elektrolytwiderstand aufweist. Dabei kann das Signal/Rausch-Verhältnis bei der Messung bis zu einem Faktor von 5 oder höher verbessert werden. Das verwendete Medium weist eine Salzkonzentration von ≤ 50 mmol/l und am meisten bevorzugt ≤ 20 mmol/l auf (angegeben als Gesamtkonzentration der im Medium vorhandenen Alkali- und Erdalkali-Kationen). Die minimale Salzkonzentration ist vorzugsweise ≥ 1 mmol/l und besonders bevorzugt ≥ 2 mmol/l. Weiterhin ist das Messmedium im Wesentlichen frei von Natriumionen, d.h. es weist eine Konzentration von Natriumionen ≤ 10 mmol/l, besonders bevorzugt ≤ 5 mmol/l und am meisten bevorzugt ≤ 1 mmol/l, auf. Ganz besonders bevorzugt bei Messung an Kaliumselektiven Ionenkanälen ist die Verwendung eines völlig von Natriumionen freien Messmediums. Auch bei der Messung an chloridselektiven oder unspezifischen Ionenkanälen ist die Verwendung eines Natrium-freien Mediums besonders bevorzugt.

Gegebenenfalls kann die Osmolalität des Messmediums durch Zugabe nichtionischer Substanzen, beispielsweise Kohlenhydrate, erhöht und auf einen Bereich zwischen 250-400 Osm, besonders bevorzugt 290-360 Osm, eingestellt werden. Die Konzentration der Kohlenhydrate im Messmedium beträgt vorzugsweise 280-340 mM.

Die Messung wird vorzugsweise als Strom-, Potenzial- oder/und Spannungsmessung am Spalt zwischen der Elektrode und der darauf immobilisierten Zelle durchgeführt. Unter Verwendung des Mediums liegt die Leitfähigkeit im Spalt bei einem Wert von ca. 10-50 $mS/cm^2$, während gemäß Stand der Technik Werte von 200-1000 $mS/cm^2$ gefunden werden.

Das Messverfahren wird vorzugsweise an einzelnen Zellen auf einer potenzialsensitiven Elektrode durchgeführt.

Ein erster Aspekt betrifft die Anwendung des salzarmen Messmedium in einem zellulären Screening-Verfahren, d.h. in einem Verfahren nach Anspruch 1 zur Bestimmung, ob eine Substanz ein Modulator eines Ionenkanal/ Rezeptorsystems in einer Zelle ist.

Das Verfahren eignet sich zur Bestimmung des Einflusses von Testsubstanzen auf beliebige Zielkomponenten in der Zelle, sofern die Zielkomponente ein an der Elektrode messbares Signal bzw. eine Änderung eines an der Elektrode messbaren Signals hervorrufen kann. Vorzugsweise bestimmt man, ob eine Substanz ein Modulator einer Membran-assoziierten Zielkomponente ist.

Die Zielkomponente kann ein oder mehrere Polypeptide umfassen. In einer besonders bevorzugten Ausführungsform umfasst die Zielkomponente ein Ionenkanal/Rezeptorsystem, umfassend eine Ionenkanal-Komponente, d.h. ein oder mehrere Polypeptide, die einen Ionentransfer, z.B. einen Transfer von Kationen und/oder Anionen, durch eine Zellmembran vermitteln können. Weiterhin umfasst das Ionenkanal/Rezeptorsystem eine RezeptorKomponente, die auf einen Stimulus reagieren kann. Bei diesem Rezeptor kann es sich um den Ionenkanal oder einen Teil des Ionenkanals handeln. Der Rezeptor kann jedoch auch ein Molekül sein, welches von dem Ionenkanal verschieden ist, das aber in operativer Verbindung zum Ionenkanal steht, d.h. eine Änderung des Funktions- und/oder Konformationsstatus des Rezeptors führt zu einer Veränderung im Funktionsstatus des Ionenkanals, wodurch eine nachweisbare Änderung des Ionenstroms durch die Zellmembran bewirkt wird. Die Stimulation des Rezeptors wird vorzugsweise durch Änderungen im Potenzial (innerhalb oder außerhalb der Zelle), durch das Vorhandensein bzw. die Anwesenheit von Effektoren, durch Beleuchtung, durch mechanische Stimulation oder Kombinationen davon bewirkt. Besonders bevorzugte Beispiele von Zielkomponenten sind spannungsgesteuerte, ligandengesteuerte und mechanisch gesteuerte Ionenkanäle, wie etwa Kaliumkanäle, z.B. die menschlichen Kaliumkanäle hSlo oder KV 1.3. Weitere bevorzugte Beispiele für Membran-assoziierte Zielkomponenten sind Rezeptoren, insbesondere NMDA, GABA, AMPA oder Acetylcholin-Rezeptoren.

Die Messung des Verfahrens wird an einer lebenden Zelle durchgeführt. Diese Zelle kann ein Mikroorganismus, z.B. eine Bakterienzelle, oder eine Hefe- oder Pilzzelle sein. Vorzugsweise ist die Zelle jedoch eine eukaryontische Zelle, besonders bevorzugt eine Säugerzelle, z.B. eine humane Zelle. Die Zelle hat vorzugsweise einen Durchmesser von $\leq 50\ \mu$m.

Weiterhin ist es bevorzugt, dass die Zelle ein oder mehrere Polypeptide überexprimiert, die die Zielkomponente oder einen Teil davon bilden oder/und die die Aktivität der Zielkomponente erhöhen. Eine derartige Zelle kann beispielsweise durch rekombinante Methoden oder Mutationen erhalten werden und zeigt eine höhere Aktivität der Zielkomponente als eine vergleichbare unbehandelte Zelle. Besonders bevorzugt ist die Zelle mit Nukleinsäure-Molekülen transfiziert, welche für die Zielkomponente kodieren. In dieser Ausführungsform umfasst die Zelle heterologe Nukleinsäure-Moleküle, die mindestens einen Teil der Zielkomponente kodieren und die eine Überexpression dieser Komponenten erlauben.

Eine bevorzugte Ausführungsform des Verfahrens umfasst die Verwendung von transgenen Zellen, die mit Nukleinsäuren kodierend für die $\alpha$- und/oder ß-Einheiten des spannungsabhängigen Kaliumkanals hSlo oder für den spannungsabhängigen Kaliumkanal KV1.3 transfiziert sind. Eine weitere bevorzugte Ausführungsform der Erfindung umfasst die Verwendung von Zellen, die mit Nukleinsäuren kodierend für den NMDA-Rezeptor, den AMPA-Rezeptor oder den GABA-Rezeptor oder einen Acetylcholin-Rezeptor transfiziert sind.

Die Zelle wird auf einer potenzialsensitiven Elektrode, insbesondere auf einer planaren potenzialsensitiven Elektrode, kultiviert. Verfahren hierzu sind beispielsweise bei Vassanelli und Fromherz (Appl. Phys. A 65 (1997), 85-88) beschrieben. Auf diese Weise kann man Zellen erhalten, die auf dem potenzialsensitiven Bereich einer Elektrode wachsen, wodurch ein operativer Kontakt zwischen der Zelle und der Elektrode resultiert.

Die Elektrodenoberfläche, auf welcher die Zelle immobilisiert ist, kann Siliciumoxid, ein anderer Halbleiter oder ein Metall sein. Die Elektrode kann auf einem Chip integriert sein, wobei der Chip vorzugsweise einen Transistor enthält. Besonders bevorzugt weist der Chip mindestens einen integrierten Feldeffekt-Transistor auf, der Source und Drain enthält. Die potenzialsensitive Elektrode kann jedoch auch eine auf einem Chip integrierte Metallelektrode sein. Vorzugsweise wird ein Transistor oder die Elektrode durch die Adhäsionsfläche der Zelle vollkommen abgedeckt.

In einer bevorzugten Ausführungsform des Verfahrens wird ein Array bereitgestellt, der eine. Vielzahl von auf jeweils verschiedenen extrazellulären potenzialsensitiven Elektroden immobilisierten Zellen umfasst. Ein solcher Array mit beispielsweise 10 oder mehr, insbesondere 50 oder mehr Zellen kann zu einem Hoch-Durchsatz-Screening verwendet werden, wobei eine Vielzahl von Testsubstanzen parallel bestimmt wird.

Die funktionellen Charakteristika der Zielkomponente in der Zelle umfassen das Entstehen eines messbaren Signals an der Elektrode. Wenn es sich bei der Zielkomponente beispielsweise um einen Ionenkanal handelt, bewirkt dessen Öffnung das Fließen eines Ionenstroms aus der Zelle oder/und in die Zelle. Dieser Ionenstrom fließt auch im Bereich des operativen Kontakts zwischen der Zelle und der Elektrode, wodurch ein nachweisbares Signal resultiert, das durch die extrazelluläre potenzialsensitive Elektrode gemessen werden kann. Das nachweisbare Signal kann beispielsweise ein Spannungsabfall im Spalt zwischen Zelle und Elektrode oder die Änderung des Oberftächenpotenzials der Elektrode aufgrund diffuser Ionenkonzentrationsänderungen im operativen Kontaktbereich sein.

Eine Änderung der funktionellen Charakteristika der Zielkomponente, z.B. der Leitfähigkeit des Ionenkanals, führt zu einer Änderung im Ionenstrom und daher in dem durch die Elektrode nachweisbaren elektrischen Signal. Das Vorhandensein eines Modulators der Zielkomponente führt ebenfalls zu einer Änderung deren funktioneller Charakteristika im Vergleich zu einer Kontrollbestimmung in Abwesenheit des Modulators. Wenn z.B. ein Ionenkanal auf einen Modulator reagiert, führt dies zu einer Änderung im Ionenstrom und somit zu einer nachweisbaren Signaländerung.

Das Verfahren umfasst vorzugsweise eine Anregung der Zielkomponente, um ein Signal an der Elektrode zu erzeugen, dessen Modulation durch die Testsubstanz nachgewiesen werden soll. Zielkomponenten, beispielsweise Ionenkanäle, können durch verschiedene Methoden, z.B. durch Anlegen einer Spannung über die Membran, durch intrazellulär und/oder extrazellulär wirkende Liganden oder durch mechanische Änderungen oder Kombinationen davon, angeregt werden. Spannungsgesteuerte Ionenkanäle ändern ihre Leitfähigkeit abhängig vom Spannungsabfall über die Membran. Durch Anlegen einer äußeren Spannung, z.B. einer Gleich- oder Wechselspannung, kann die Leitfähigkeit von spannungsgesteuerten Ionenkanälen moduliert werden. Gegebenenfalls kann hierzu noch eine zusätzliche Elektrode, z.B. eine Patchclamp-Elektrode, verwendet werden, die in die Zelle eingebracht ist oder unmittelbar mit ihr im Kontakt steht. Auf diese Weise kann der Spannungsabfall auf einen festen Wert eingestellt werden.

Die Zielkomponente kann auch durch einen Liganden angeregt werden. Bei ligandengesteuerten Ionenkanälen existieren zwei Mechanismen, nämlich ionotropische und Second-Messenger-Systeme. In ionotropischen Systemen binden Ligandenmoleküle direkt an die Zielkomponente und ändern deren Charakteristika, beispielsweise können intrazelluläre Calcium-Ionen die Leitfähigkeit von Kaliumkanälen beeinflussen. In Second-Messenger-Systemen binden die Ligandenmoleküle an einen Rezeptor, der erst über eine Signalkaskade den Ionenkanal beeinflusst.

Selbstverständlich können diese verschiedenen Methoden zur Anregung der Zielkomponenten auch kombiniert werden, um ein wirksames Messsystem für den Einfluss der Änderung von Umgebungsparametern, z.B. von Testsubstanzen, auf eine Zielkomponente in einer Zelle bereitzustellen.

Ein weiterer Aspekt ist ein bioelektronisches System nach Anspruch 12.

Das bioelektrische System kann zur Messung von durch die Zielkomponente hervorgerufenen Signalen an der potenzialsensitiven Zielkomponente hervorgerufenen Signalen an der potenzialsensitiven Elektrode verwendet werden. Vorzugsweise wird eine Analyse an Einzelzellen durchgeführt, wobei die Lokalisierung oder/und die Charakteristika, z.B. die Eigenschaften eines Ionenkanals an einer einzelnen Zelle, bestimmt werden können. Somit eignet sich die bioelektronische Vorrichtung als Sensor, z.B. zur Bestimmung der Änderung eines Umgebungsparameters als nachweisbares Signal an der Elektrode. Insbesondere eignet sich die Vorrichtung zur Bestimmung, ob eine Substanz ein Modulator, z.B. ein Aktivator oder Inhibitor, der Zielkomponente in der Zelle ist.

Ein weiterer Aspekt ist ein Verfahren zur Lokalisierung einer Zelle auf einem Array, nach Anspruch 18.

Hierbei umfasst der Array bevorzugt eine Vielzahl extrazellulärer potenzialsensitiver Elektroden. Vorteilhafterweise wird bei diesem Verfahren eine ortspezifische Messung durchgeführt.

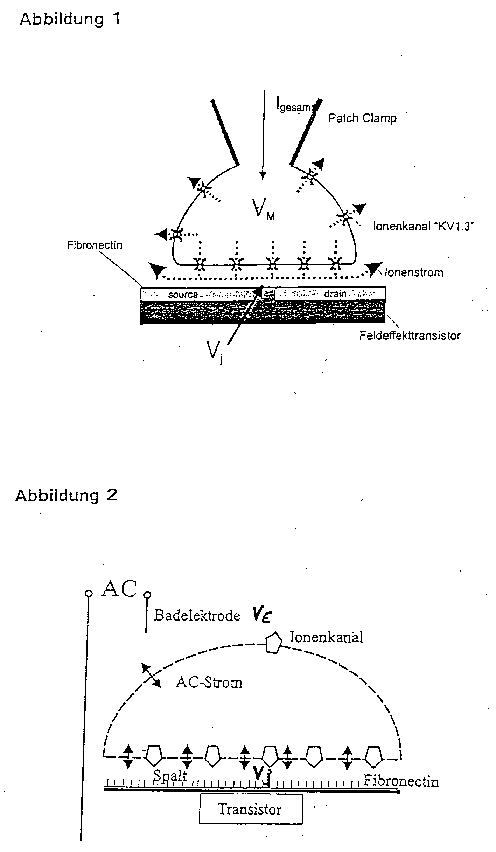

Im Folgenden werden zwei bevorzugte Ausführungsformen der vorliegenden Erfindung näher erläutert. Die erste Ausführungsform ist in **Abbildung 1** dargestellt und zeigt eine auf einem Feldeffekt-Transistor mit Drain, Source und Gate immobilisierte Zelle, die in ihrer Membran einen Ionenkanal, z.B. den KV1.3-Kaliumkanal, enthält. Die Oberfläche der Elektrode ist mit Fibronectin beschichtet. In die Zelle ist weiterhin eine Patch-Elektrode zur Einstellung eines definierten Potenzials eingeführt. Bei dieser Ausführungsform werden die in der Zellmembran lokalisierten Kanäle durch Anlegen einer Gleichspannung angeregt. Der Spannungsabfall in der potenzialsensitiven Elektrode $V_j$ ist durch die spezifische Leitfähigkeit der adhärierten Membran $g_{jm}$ sowie durch die spezifische Leitfähigkeit des Spaltes $g_j$ gegeben:

$$V_j \quad = \quad \frac{g_{jm}}{g_{jm} + g_j}(V_m - V_o)$$

wobei $V_m$ die intrazelluläre Spannung und $V_o$ das Nernstpotenzial der Zelle ist.

Die zweite Ausführungsform betrifft die Messung von Zielkomponenten, z.B. geschlossenen bzw. geöffneten Ionenkanälen, bei Anlegen von Wechselspannung. Diese Ausführungsform ist in Abbildung 2 gezeigt.

Das Verhältnis zwischen der anregenden Spannung $V_E$ (im Medium) und der vom Transistor gemessenen Spannung im Spalt $V_j$ kann durch folgende Formel bestimmt werden:

$$h \quad = \quad \frac{V_j}{V_E} \quad = \quad \frac{1 + i\omega\tau_j h_\infty}{1 + i\omega\tau_j} \, ,$$

wobei

$$h_\infty \quad = \quad \frac{c_m}{c_m + (1 + \beta)c_{ox}}$$

und

$$\tau_j \quad = \quad \frac{c_m + (1 + \text{ß})c_{ox}}{g_m + (1 + \text{ß})g_j}$$

$\tau_j$ ist die charakteristische Zeitkonstante der Übertragungsfunktion $V_j/V_E$,

$h_\infty$ ist das Verhältnis $V_j/V_E$ im Grenzfall hoher Frequenzen,

$\omega$ ist die Frequenz $*2*\pi$,

$c_m$ ist die spezifische Membrankapazität,

$c_{ox}$ ist die spezifische Oxidkapazität der Chipoberfläche,

ß ist das Flächenverhältnis der oberen zur der adhärierten Membran,

$g_m$ ist die spezifische Membranleitfähigkeit,

$g_j$ ist die spezifische Leitfähigkeit im Spalt.

Ist $g_j$ in der Größenordnung von $g_m$, so kann eine Änderung der Membranleitfähigkeit (z.B. durch das Öffnen von ligandengesteuerten Kanälen in der Zellmembran) in der Phase sowie in der Amplitude der Übertragungsfunktion gemessen werden. Eine Senkung von $g_j$ durch die neue Elektrolytzusammensetzung erreicht diesen Zweck. In der bisherigen Elektrolytzusammensetzung ist die Membranleitfähigkeit vernachlässigbar und eine Änderung dieser in der Übertragungsfunktion nicht mehr messbar.

Die Vorrichtung und das Verfahren eignen sich zur Bestimmung einer Veränderung von Umgebungsparametern als nachweisbares Signal an der Elektrode. Insbesondere eignet sich das Verfahren zur Bestimmung, ob eine Testsubstanz die Rezeptorkomponente eines Ionenkanal/Rezeptorsystems aktivieren oder inhibieren kann. Die Rezeptorkomponente kann ein pharmazeutisch relevantes Zielmolekül sein. Beispielsweise kann die Wirkung von Testsubstanzen auf die Freisetzung von Neurotransmittern untersucht werden.

In einer weiteren Ausführungsform eignet sich das Verfahren zum Nachweis des Vorhandenseins oder der Menge einer Substanz, die als Effektor auf die Rezeptorkomponente wirkt.

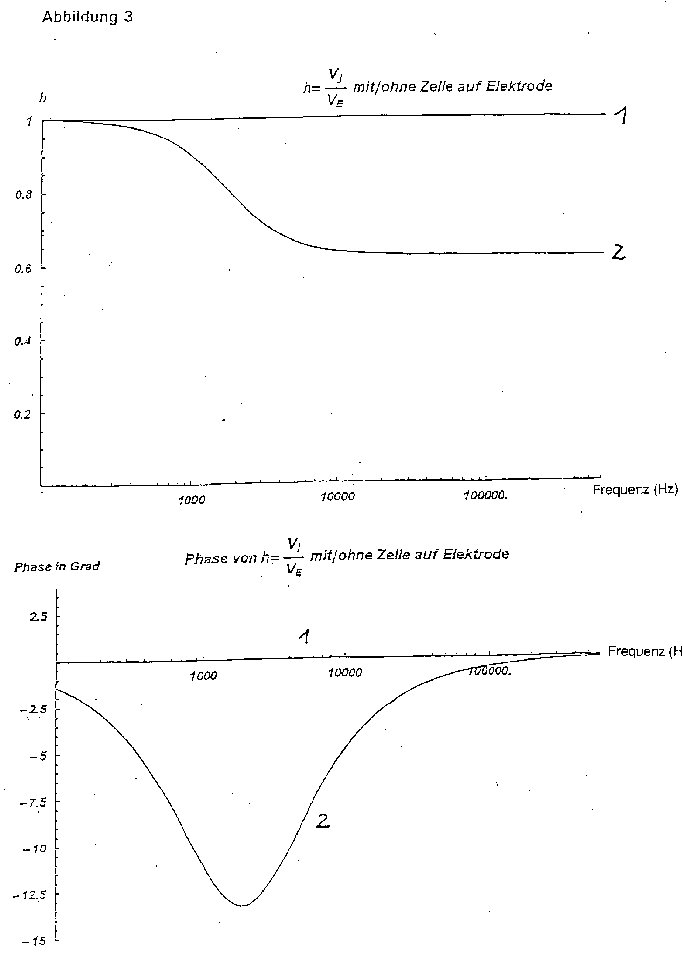

Bei der Verwendung eines Elektrodenarrays kann die Übertragungsfunktion $V_j/V_E$ zur Lokalisierung von immobilisierten Einzelzellen benutzt werden (siehe Abbildung 3). Dabei wird pro Elektrode vorzugsweise eine Einzelzelle getestet. In **Abbildung 3A** zeigt Kurve (1) den Verlauf von h ohne Zelle, während Kurve (2) den Verlauf von h mit Zelle zeigt. In **Abbildung 3B** ist die Phase von h ohne Zelle als Kurve (1) und mit Zelle als Kurve (2) gezeigt.

Weiterhin soll die Erfindung durch die nachfolgenden Beispiele erläutert werden.

1. Materialien und Methoden

1.1 Plasmide und Zellen

HEK293-Zellen wurden durch die Calciumphosphat-Methode stabil mit einem Plasmid transfiziert, das durch Klonierung einer für den humanen KV1.3-Kaluimkanal kodierenden Nukleinsäure in den Vektor pcDNA3 (Invitrogen) erhalten wurde.

1.2 Zellkultur auf dem Substrat

Die transfizierten Zellen wurden auf einen Fibronectin-beschichteten Siliciumchip mit 62 Feldeffekt-Transistoren aufgebracht. Nach 1 Tag in Kultur wird die Nährflüssigkeit durch eine physiologische Extrazellulärlösung (135 mM NaCl, 5 mM KCl, 1,8 mM $CaCl_2$, 1 mM $MgCl_2$, Osmolalität mit Glucose auf 340 mOsmol/kg ausgeglichen, pH-Wert 7,4) ausgetauscht. Gegebenenfalls können Zellen mit Patchclamp-Elektroden versehen werden.

**Abbildung 4** zeigt eine transfizierte Zelle, die auf dem Gate eines EOSFET (Electrolyte Oxide Silicon Field Effect Transistor) adhäriert ist, vor (A) und nach (B) der Messung durch eine Patchclamp.

**Abbildung 5** zeigt die Wirkung physiologischer Extrazellulärlösung. Die Spannung in der Zelle V, der Strom in der Zelle I sowie die im Spalt abfallende Gate-Spannung $V_j$ sind gegen die Zeit aufgetragen. Die Pulslänge beträgt 50 ms. Die Messungen sind im Spannungs-Clamp-Modus durchgeführt. Der Strom zeigt das typische zeitliche Verhalten eines KV1.3-Kanals.

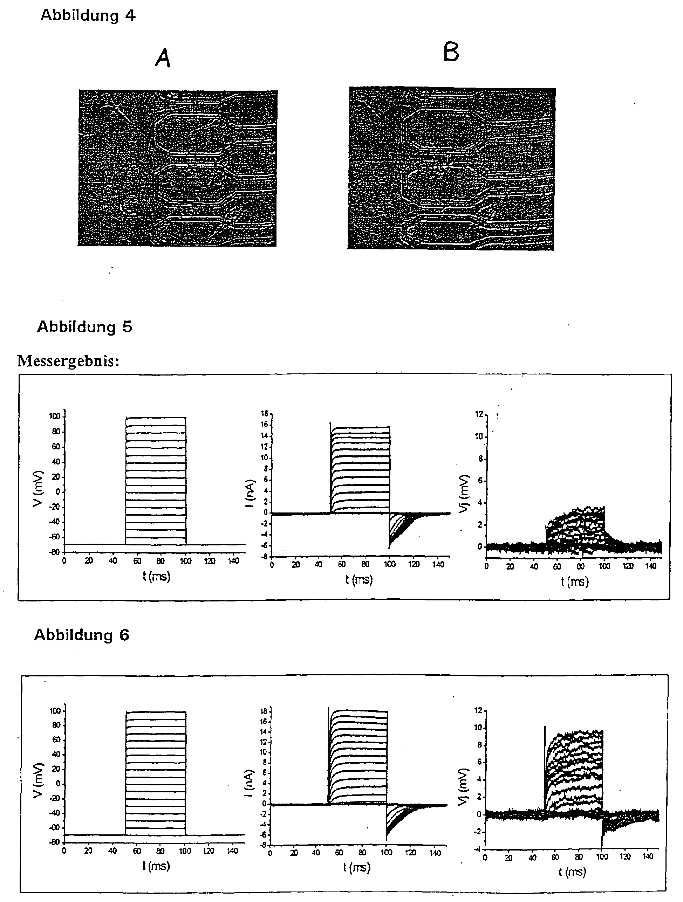

Wird nun die physiologische Extrazellulärlösung durch eine NaCl-freie Lösung (0 mM NaCl, 5 mM KCl, 1,8 mM $CaCl_2$, 1 mM $MgCl_2$, Osmolalität mit Glucose auf 340 mOsmol/kg ausgeglichen, pH-Wert 7,4) ausgetauscht, so ergibt dies die in **Abbildung** 6 gezeigte Messung. Der Austausch der Lösung führt zu einer signifikanten Verbesserung des Signal/Rausch-Verhältnisses durch eine Signalerhöhung um den Faktor 4. Das Verhalten der Ionenkanäle wird durch

den Lösungstausch nicht beeinflusst (sowohl Maximalstrom als auch das zeitliche Verhalten bleiben unverändert), zudem nehmen die Zellen keinen beobachtbaren Schaden.

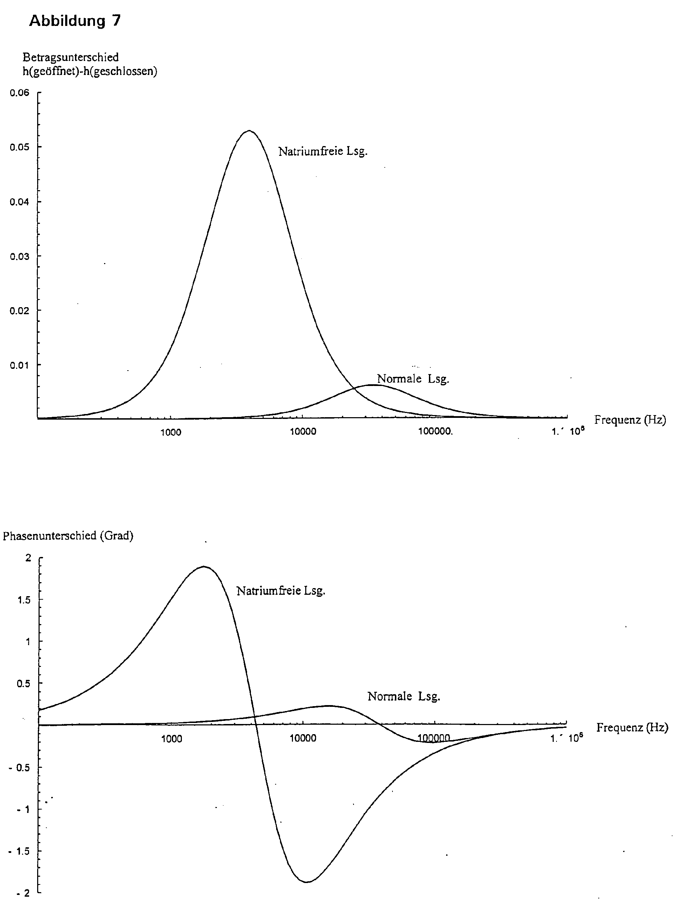

Der Unterschied der Messungen bei normalen Lösungen und natriumfreien Lösungen nach Anregung durch Wechselspannung ist in **Abbildung 7** gezeigt.

**Patentansprüche**

1. Verfahren zur Bestimmung, ob eine Substanz ein Modulator eines Ionenkanal/Rezeptorsystems in einer Zelle ist, umfassend die Schritte:

    (a) Bereitstellen einer auf einer planaren extrazellulären potenzialsensitiven Elektrode immobilisierten transgenen Zelle, die das Ionenkanal/Rezeptorsystem enthält, wodurch ein operativer Kontakt zwischen der Zelle und der Elektrode resultiert,
    (b) Inkontaktbringen einer zu testenden Substanz mit der Zelle in einem Medium, das eine Gesamtkonzentration von Alkali- und Erdalkali-Kationen von $\leq 50$ mmol/l aufweist,
    (c) Messen einer durch das Ionenkanal/Rezeptorsystem hervorgerufenen Signaländerung an der Elektrode aufgrund eines Ionenstroms im Bereich des operativen Kontakts zwischen der Zelle und der Elektrode, und
    (d) Bestimmen des Einflusses der zu testenden Substanz auf das Messsignal.

2. Verfahren nach Anspruch 1, **dadurch gekennzeichnet, dass** Ionenkanal/Rezeptorsystem ein Membran-assoziiertes Ionenkanal/Rezeptorsystem ist.

3. Verfahren nach Anspruch 1 oder 2, **dadurch gekennzeichnet, dass** das Ionenkanal-Rezeptorsystem einen spannungsgesteuerten, ligandengesteuerten oder mechanisch gesteuerten Ionenkanal enthält.

4. Verfahren nach einem der vorhergehenden Ansprüche, **dadurch gekennzeichnet, dass** der Ionenkanal ein Kaliumkanal ist.

5. Verfahren nach einem der vorhergehenden Ansprüche, **dadurch gekennzeichnet, dass** der Kaliumkanal ausgewählt wird aus hSlo und KV1.3.

6. Verfahren nach Anspruch 4, **dadurch gekennzeichnet, dass** das Ionenkanal-Rezeptorsystem einen NMDA, GABA, AMPA oder Acetylcholin-Rezeptor enthält.

7. Verfahren nach einem der vorhergehenden Ansprüche weiterhin umfassend ein Anregen des Ionenkanal/Rezeptorsystems in der Zelle, **dadurch gekennzeichnet, dass** das Anregen des Ionenkanal/Rezeptorsystems eine elektrische, optische oder/und chemische Anregung umfasst.

8. Verfahren nach Anspruch 7, **dadurch gekennzeichnet, dass** das Anregen des Ionenkanal/Rezeptorsystems das Anlegen einer Gleichspannung oder einer Wechselspannung umfasst.

9. Verfahren nach einem der vorhergehenden Ansprüche, **dadurch gekennzeichnet, dass** die Zelle mit einer zusätzlichen Elektrode, z. B. einer Patchclamp in Kontakt steht.

10. Verfahren nach einem der vorhergehenden Ansprüche, **dadurch gekennzeichnet, dass** die potenzial-sensitive extrazelluläre Elektrode auf einem Chip angeordnet ist.

11. Verfahren nach einem der vorhergehenden Ansprüche, **dadurch gekennzeichnet, dass** man einen Array, umfassend eine Vielzahl von auf verschiedenen Elektroden immobilisierten Zellen bereitstellt und eine Vielzahl von Substanzen testet.

12. Bioelektronisches System, umfassend

    - eine transgene Zelle, die ein Ionenkanal/Rezeptorsystem enthält,
    - eine planare potenzialsensitive extrazelluläre Elektrode, wobei die Zelle auf der planaren potenzialsensitiven extrazellulären Elektrode immobilisiert ist und das Ionenkanal/Rezeptorsystem ein an der Elektrode messbares Signal aufgrund eines Ionenstroms im Bereich eines operativen Kontakts zwischen der Zelle und der Elektrode hervorrufen kann, und

- ein Medium mit einer Gesamtkonzentration von Alkali- und Erdalkali-Kationen von $\leq$ 50 mmol/l in Kontakt mit der Zelle und der Elektrode.

**13.** Verwendung des bioelektrischen Systems nach Anspruch 12 zur Messung von durch das Ionenkanal/Rezeptorsystem hervorgerufenen Signalen an der potenzialsensitiven Elektrode.

**14.** Verwendung nach Anspruch 13 zur Analyse von Einzelzellen.

**15.** Verwendung nach Anspruch 13 oder 14 als Sensor zur Bestimmung der Änderung eines Umgebungsparameters als nachweisbares Signal an der Elektrode.

**16.** Verwendung nach einem der Ansprüche 13 bis 15, **dadurch gekennzeichnet, dass** die Änderung eines Umgebungsparameters als nachweisbares Signal an der Elektrode bestimmt wird.

**17.** Verwendung nach einem der Ansprüche 13 bis 16 zur Bestimmung, ob eine Substanz ein Modulator des Ionenkanal/Rezeptorsystems ist.

**18.** Verfahren zur Lokalisierung einer auf einem Array immobilisierten Einzelzelle, wobei der Array eine Vielzahl extrazellulärer potenzialsensitiver Elektroden umfasst, umfassend die Schritte:

(a) Bereitstellen einer auf einer planaren extrazellulären potenzialsensitiven Elektrode immobilisierten transgenen Einzelzelle, die ein Ionenkanal/Rezeptorsystem enthält, wodurch ein operativer Kontakt zwischen der Zelle und der Elektrode resultiert,

(b) Messen eines Signals an der Elektrode, hervorgerufen durch die Anwesenheit der Zelle, in einem Medium, das eine Gesamtkonzentration von Alkali- und Erdalkali-Kationen von $\leq$ 50 mmol/l aufweist, und

(c) Lokalisieren der Position der immobilisierten transgenen Einzelzelle durch die Messung in (b) und worin Schritt (b) eine ortsspezifische Messung dadurch umfasst, dass Signale von Elektroden mit immobilisierter Einzelzelle und Elektroden ohne immobilisierte Einzelzelle gemessen werden.

## Claims

**1.** A method for determining whether a substance is a modulator of an ion channel/receptor system in a cell, comprising the steps of:

(a) providing a transgenic cell immobilized on a planar extracellular potential-sensitive electrode, which contains the ion channel/receptor system, whereby an operative contact results between the cell and the electrode;

(b) bringing a substance to be tested into contact with the cell in a medium, which has a total concentration of alkali and alkaline-earth cations of $\leq$ 50 mmol/l,

(c) measuring a signal change caused by the ion channel/receptor system at the electrode due to an ion current in the region of the operative contact between the cell and the electrode; and

(d) determining the effect of the substance to be tested on the measurement signal.

**2.** The method according to Claim 1, **characterized in that** the ion channel/receptor system is a membrane-associated ion channel/receptor system.

**3.** The method according to Claim 1 or 2, **characterized in that** the ion channel/receptor system contains a voltage-controlled, ligand-controlled or mechanically controlled ion channel.

**4.** The method according to any one of the preceding claims, **characterized in that** the ion channel is a potassium channel.

**5.** The method according to any one of the preceding claims, **characterized in that** the potassium channel is selected from hSlo and KV1.3.

**6.** The method according to Claim 4, **characterized in that** the ion channel/receptor system contains an NMDA, GABA, AMPA or acetylcholine receptor.

7. The method according to any one of the preceding claims, further comprising an excitation of the ion channel/receptor system in the cell, **characterized in that** the excitation of the ion channel/receptor system comprises an electrical, optical and/or chemical excitation.

8. The method according to Claim 7, **characterized in that** the excitation of the ion channel/receptor system comprises the application of a DC voltage or an AC voltage.

9. The method according to any one of the preceding claims, **characterized in that** the cell is in contact with an additional electrode, for example, a patch clamp.

10. The method according to any one of the preceding claims, **characterized in that** the potential-sensitive extracellular electrode is arranged on a chip.

11. The method according to any one of the preceding claims, **characterized in that** an array comprising a plurality of cells immobilized on different electrodes is provided, and a plurality of substances is tested.

12. A bioelectronic system, comprising:

- a transgenic cell which contains an ion channel/receptor system;
- a planar potential-sensitive extracellular electrode, wherein the cell on the planar potential-sensitive extracellular electrode is immobilized, and the ion channel/receptor system can cause a signal measurable at the electrode due to an ion current in the region of an operative contact between the cell and the electrode; and
- a medium having a total concentration of alkali and alkaline-earth cations of $\leq 50$ mmol/l in contact with the cell and the electrode.

13. Use of the bioelectronic system according to Claim 12 for measuring signals caused by the ion channel/receptor system at the potential-sensitive electrode.

14. Use according to Claim 13 for analyzing individual cells.

15. Use according to Claim 13 or 14 as a sensor for determining the change in an ambient parameter as a detectable signal at the electrode.

16. Use according to any one of Claims 13 to 15, **characterized in that** the change in an ambient parameter is determined as a detectable signal at the electrode.

17. Use according to any one of Claims 13 to 16 for determining whether a substance is a modulator of the ion channel/receptor system.

18. A method for localizing an individual cell immobilized on an array, wherein the array comprises a plurality of extracellular potential-sensitive electrodes, comprising the steps of:

(a) providing a transgenic individual cell immobilized on a planar extracellular potential-sensitive electrode, which contains an ion channel/receptor system, whereby an operative contact results between the cell and the electrode;
(b) measuring a signal at the electrode caused by the presence of the cell, in a medium which has a total concentration of alkali and alkaline-earth cations of $\leq 50$ mmol/l; and
(c) localizing the position of the immobilized transgenic individual cell through the measurement in (b), and wherein step (b) comprises a location-specific measurement in that signals from electrodes with an immobilized individual cell and electrodes without an immobilized individual cell are measured.

**Revendications**

1. Procédé pour déterminer si une substance est un modulateur d'un système de canal ionique/récepteur dans une cellule, comprenant les étapes suivantes :

(a) mise à disposition d'une cellule transgénique immobilisée sur une électrode extracellulaire plane sensible à un potentiel qui contient le système de canal ionique/récepteur, ce qui entraîne un contact opérationnel entre la cellule et l'électrode,

(b) mise en contact d'une substance à tester avec la cellule dans un milieu qui présente une concentration totale de cations alcalins et alcalinoterreux de ≤ 50 mmol/l,

(c) mesure d'une modification de signal provoquée par le système de canal ionique/récepteur sur l'électrode en raison d'un flux d'ions dans la zone du contact opérationnel entre la cellule et l'électrode, et

(d) détermination de l'influence de la substance à tester sur le signal de mesure.

**2.** Procédé selon la revendication 1, **caractérisé en ce que** le système de canal ionique/récepteur est un système de canal ionique/récepteur associé à une membrane.

**3.** Procédé selon la revendication 1 ou 2, **caractérisé en ce que** le système de canal ionique/récepteur contient un canal ionique commandé par un courant, commandé par un ligand ou commandé mécaniquement.

**4.** Procédé selon l'une des revendications précédentes, **caractérisé en ce que** le canal ionique est un canal potassique.

**5.** Procédé selon l'une des revendications précédentes, **caractérisé en ce que** le canal potassique est choisi parmi hSio et KV1.3.

**6.** Procédé selon la revendication 4, **caractérisé en ce que** le système de canal ionique/récepteur contient un récepteur NMDA, GABA, AMPA ou acétylcholine.

**7.** Procédé selon l'une des revendications précédentes, comprenant en outre une excitation du système de canal ionique/récepteur dans la cellule, **caractérisé en ce que** l'excitation du système de canal ionique/récepteur comprend une excitation électrique, optique et/ou chimique.

**8.** Procédé selon la revendication 7, **caractérisé en ce que** l'excitation du système de canal ionique/récepteur comprend l'application d'un courant continu ou d'un courant alternatif.

**9.** Procédé selon l'une des revendications précédentes, **caractérisé en ce que** la cellule est en contact avec une électrode supplémentaire, par exemple un patch-clamp.

**10.** Procédé selon l'une des revendications précédentes, **caractérisé en ce que** l'électrode extracellulaire sensible à un potentiel est disposée sur une puce.

**11.** Procédé selon l'une des revendications précédentes, **caractérisé en ce que** l'on met à disposition un agencement comprenant une multiplicité de cellules immobilisées sur des électrodes différentes et l'on teste une multiplicité de substances.

**12.** Système bio-électronique, comprenant

- une cellule transgénique qui contient un système de canal ionique/récepteur,
- une électrode extracellulaire plane sensible à un potentiel, dans lequel la cellule est immobilisée sur l'électrode extracellulaire plane sensible à un potentiel et le système de canal ionique/récepteur peut provoquer un signal mesurable sur l'électrode en raison d'un flux d'ions dans la zone d'un contact opérationnel entre la cellule et l'électrode, et
- un milieu qui présente une concentration totale de cations alcalins et alcalinoterreux de ≤ 50 mmol/l en contact avec la cellule et l'électrode.

**13.** Utilisation du système bio-électrique selon la revendication 12 pour mesurer des signaux provoqués par le système de canal ionique/récepteur sur l'électrode sensible à un potentiel.

**14.** Utilisation selon la revendication 13 pour l'analyse de cellules individuelles.

**15.** Utilisation selon la revendication 13 ou 14 comme capteur pour déterminer la modification d'un paramètre environnemental en tant que signal détectable sur l'électrode.

**16.** Utilisation selon l'une des revendications 13 à 15, **caractérisée en ce que** la modification d'un paramètre environnemental en tant que signal détectable est déterminée sur l'électrode.

**17.** Utilisation selon l'une des revendications 13 à 16, pour déterminer si une substance est un modulateur du système de canal ionique/récepteur.

**18.** Procédé de localisation d'une cellule individuelle immobilisée sur un agencement, dans lequel l'agencement comprend une multiplicité d'électrodes extracellulaires sensibles à un potentiel, comprenant les étapes suivantes :

(a) mise à disposition d'une cellule transgénique individuelle immobilisée sur une électrode extracellulaire plane sensible à un potentiel qui contient le système de canal ionique/récepteur, ce qui entraîne un contact opérationnel entre la cellule et l'électrode,

(b) mesure d'un signal sur l'électrode, provoqué par la présence de la cellule dans un milieu qui présente une concentration totale de cations alcalins et alcalinoterreux de $\leq 50$ mmol/l, et

(c) localisation de la position de la cellule transgénique individuelle immobilisée par la mesure du point (b) et où l'étape (b) comprend une mesure spécifique à un site de telle sorte que des signaux d'électrodes comportant une cellule individuelle immobilisée et d'électrodes sans cellule individuelle immobilisée soient mesurés.

Abbildung 1

Abbildung 2

Abbildung 3

$$h = \frac{V_J}{V_E} \text{ mit/ohne Zelle auf Elektrode}$$

$$\text{Phase von } h = \frac{V_J}{V_E} \text{ mit/ohne Zelle auf Elektrode}$$

Abbildung 4

A          B

Abbildung 5

Messergebnis:

Abbildung 6

**Abbildung 7**

Betragsunterschied
h(geöffnet)-h(geschlossen)

Natriumfreie Lsg.

Normale  Lsg.

Frequenz (Hz)

1000          10000          100000.          1.′ 10⁶

Phasenunterschied (Grad)

Natriumfreie Lsg.

Normale  Lsg.

Frequenz (Hz)

1000          10000          100000          1.′ 10⁶

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5827655 A
- WO 0194375 A1
- US 20020086298 A1
- EP 0103209 W
- WO 9631774 A
- US PS6051422 A, Baumann

**In der Beschreibung aufgeführte Nicht-Patentliteratur**

- *Sensors and Activators,* 1999, vol. B55, 77-89
- **FERTIG et al.** *Biophys. J.,* 2002, vol. 82, 3056-3062
- **VASSANELLI ; FROMHERZ.** *Appl. Phys. A,* 1997, vol. 65, 85-88